# EUROPEAN PATENT APPLICATION

(11) **EP 4 814 487 A1**
(43) Date of publication of application: **30.09.2026**
(21) Application number: 25382289.4
(22) Date of filing: 26.03.2025
(51) Int. Cl.: G01N 33/487, A61B 17/425, C12M 1/34, C12N 5/071, G01N 15/14, G01N 33/68

(54) **ELECTROMECHANICAL-OPTICAL MICROPIPETTE FOR IN-SITU IN VITRO GAMETE/EMBRYO ANALYSIS AND INJECTION**

(71) Applicant: ASOCIACIÓN CENTRO DE INVESTIGACIÓN COOPERATIVA EN NANOCIENCIAS "CIC nanoGUNE", 20018 San Sebastian, Guipuzcoa (ES)
(72) Inventor: MEDINA SÁNCHEZ, Mariana, E-20018 San Sebastián - Guipúzcoa (ES)
(74) Representative: Icosa

(57) **Abstract**

The present invention relates to a system for assessing a germinal cell (210) and/or an embryonic cell (210) in an in vitro procedure, the system comprising a pipette (100), optionally a Petri dish, at least one sensor (150) configured to measure a physical parameter of the germinal cell (210) and/or embryonic cell (210) and at least one computing unit configured to assess, based on the physical parameter, the germinal cell (210) and/or embryonic cell (210).

## Description

### FIELD OF INVENTION

The present invention relates to a system and method for assessing a germinal cell and/or an embryonic cell in an *in vitro* procedure.

### BACKGROUND OF INVENTION

Assessment of oocytes and sperm cells occurs before intracytoplasmic sperm injection and plays an important role in improving oocyte fertilization and generating embryos with higher implantation and developmental potential.

With known methods, oocyte and sperm assessments are mainly based on motility and concentration of sperm cells. However, analysis of motility to assess sperm cells lacks precision and relies on a representative sample concentration which is not always representative of the sperm that will be used for fecundation.

A deeper analysis of both oocyte and sperm cells is thus needed to understand factors influencing pregnancy chances (whether successful or not) and to allow to predict fecundation and pregnancy viability.

Current technologies, particularly those involving multiparametric analysis beyond sperm motility and concentration, rely on biochemical assays that are destructive. Moreover, these assays rely on statistical assessments from a representative sample sent to an external lab, which can take days or weeks for results. By the time the fresh sperm sample is analyzed, its characteristics may have changed, making the analysis less representative. Significant variability and heterogeneity within semen samples can make analysis and sperm selection quite arbitrary.

There is thus a need for a system allowing to assess oocytes and/or sperm cells beyond motility and concentration parameters which is non-destructive and which is not based on statistical assessments.

Moreover, after *in vitro* fertilization, assessment of embryonic cells plays an important role in improving the success of pregnancy by diagnosing genetic defects before embryo implantation.

Currently known preimplantation genetic diagnosis (PGD) involves embryo biopsy. One or two cells are extracted from an embryo on Day 5 or Day 6 for chromosomal abnormalities analysis. However, the removal of cells from the embryo could potentially harm the embryo or reduce the chances of implantation. Some studies suggest that embryo biopsy may affect placental development. Moreover, PGD may provide false results. Finally, PGD targets a specific set of genetic diseases. Embryo biopsy is not always feasible for every embryo. It can be costly and, in some countries, is prohibited. As a result, parents may face difficult decisions, such as considering an abortion after three months if a genetic disease is discovered that they are not prepared to manage.

There is thus a need for a system that allows for the minimally invasive or non-destructive *in situ* assessment of gametes or embryo cells, evaluating a wide range of parameters - including genetics, metabolism, morphology, and function - within a short timeframe.

To this aim, the invention relates to a system for assessing a germinal cell and/or an embryonic cell in an in vitro procedure, the system comprising a pipette and at least one sensor configured to measure a physical parameter of the germinal cell and/or embryonic cell.

### SUMMARY

This invention thus relates to a system for assessing a germinal cell and/or an embryonic cell in an in vitro procedure, the system comprising
- a pipette configured to hold at least one germinal cell and/or an embryonic cell;
- at least one sensor configured to measure a physical parameter of the germinal cell and/or embryonic cell;
- at least one computing unit configured to assess, based on the physical parameter, the germinal cell and/or embryonic cell.

This system allows embryologists to perform more precise assessments of the quality of germinal cells used in *in vitro* fertilization (IVF) and of embryos issued from IVF. Indeed, the sensor directly measures parameters of the cells and its culturing media without having the need to wait for a laboratory to provide the results. For example, sperm cells or oocytes selected for the fecundation are analyzed just before fecundation allowing to determine if the gamete is in optimal condition for fertilization. The system allows for a non-destructive assessment which is not based on statistical sample. Moreover, the system allows embryos to assess without cell extraction. Finally, this system allows multiparametric analysis and is compatible with currently used intracytoplasmic sperm injection (ICSI) and in vitro fertilization (IVF) systems. Therefore, a wide range of parameters may be measured. This thus provides a fast and simple way to assess the quality of germinal cells and embryos to increase the chances of a successful pregnancy.

According to an advantageous embodiment, the sensor is a strain gauge sensor disposed on an external surface of the pipette and the physical parameter is a strain of the germinal cell and/or embryonic cell.

This allows to measure deformation of the germinal/embryonic cells at the nanometer or micrometer scale. The deformation may be used to calculate the Young modulus of the germinal/embryonic cells. This parameter helps assessing the quality of gametes/embryos.

According to an advantageous embodiment, the sensor is configured to apply pressure on a surface of the germinal cell and/or embryonic cell and the physical parameter is the elastic deformation of said surface, the computing unit being configured to compute a mechanobiology parameter, preferably Young modulus, from the measured elastic deformation.

The mechanobiology parameter helps assessing the quality of gametes/embryos.

According to an advantageous embodiment, the sensor comprises a microspring, and/ or a surface acoustic wave generator.

The surface acoustic wave generator generates acoustic waves that propagate and reflect on the germinal/embryonic cells. A measure of the resulting acoustic waves allows to determine the elastic deformation of the germinal cell and/or embryonic cell. The elastic deformation may also be determined by using a microspring used to apply a pressure on the cell held by the pipette.

According to an advantageous embodiment, the pipette comprises a constriction channel and the system further comprises a vacuum generator configured to generate a suction force in the constriction channel.

This allows to deform the germinal cell and/or embryonic cell held by the pipette. A measure of this deformation allows for assessing the mechanical properties of the cell.

According to an advantageous embodiment, the sensor is a Raman spectrometer comprising a light source configured to emit a probe light beam towards the germinal cell and/or embryonic cell, and a microdetector configured to detect a light beam received from the germinal cell and/or embryonic cell in response to the probe light beam, the physical parameter being a spectrum of the detected light beam, the computing unit being configured to determine a DNA integrity of the germinal cell and/or embryonic cell, a presence of oxidative stress markers in the germinal cell and/or embryonic cell, information on biomolecules inside the germinal cell and/or embryonic cell, and/or mechanical properties of the germinal cell and/or embryonic cell from the spectrum.

This embodiment takes advantage of the curvature of the pipette to collimate the Raman light that is used to excite the sample. The spectrum of the light beam detected by the microdetector shaped by the expressed biomarkers, metabolites, and the germinal cells or embryo cells themselves. Therefore, this allows to assess the biological properties of the cells.

According to an advantageous embodiment, the pipette comprises gold nanoparticles so that the sensor is configured to perform Surface Enhanced Raman spectroscopy.

This allows to considerably improve the sensitivity of Raman spectroscopy to levels of single-molecule detection.

According to an advantageous embodiment, the system further comprises a petri dish configured to contain at least one germinal cell and/or an embryonic cell, the petri dish comprising the at least one sensor.

According to an advantageous embodiment, the germinal cell is a gamete, the pipette being configured to put the gamete held in the pipette in contact with another gamete for intracytoplasmic sperm injection.

According to an advantageous embodiment, the pipette is a holding pipette.

According to an advantageous embodiment, the pipette is a micropipette.

According to an advantageous embodiment, the pipette is configured to hold a sperm cell and comprises a constriction channel and/or a porous membrane disposed transversally inside the pipette and a negative pressure applicator configured to decrease a pressure inside the pipette.

This allows to secure the cell inside the pipette to avoid losing the cell during analysis. Moreover, this allows to hold the cell in a predetermined orientation relatively to the pipette. This thus allows to maintain the cell in place relatively to the sensor and therefore improves the quality of assessment of the cell.

According to an advantageous embodiment, the sensor comprises two electrodes disposed on an internal surface of the pipette configured to hold a sperm cell so that the sperm cell is positioned between the two electrodes, the physical parameter being an impedance of the sperm cell, the computing unit being configured to compute integrity of the sperm cell from the measured impedance.

This allows to measure the impedance due to the expressed biomarkers, metabolites, and the cells themselves. This allows to determine oxidative stress, expressed biomarkers and properties on the membrane and cytoplasm conductivity.

Moreover, the electrodes may be used for Surface Enhanced Raman spectroscopy.

This invention also relates to a method for assessing a germinal cell and/or an embryonic cell in an in vitro procedure, the method comprising:
- providing a system comprising:
   o a pipette;
   ∘ at least one sensor configured to measure a physical parameter of a germinal cell and/or an embryonic cell;
   ∘ at least one computing unit configured to assess, based on the physical parameter, the germinal cell and/or embryonic cell;
- providing at least one germinal cell and/or at least one embryonic cell;
- holding the germinal cell and/or the embryonic cell using the pipette;
- measuring a physical parameter of the germinal cell and/or the embryonic cell with the sensor; and
- assessing, based on the physical parameter, the at least one germinal cell and/or an embryonic cell.

According to an advantageous embodiment, the germinal cell may be a sperm cell or an oocyte and the embryonic cell may be a zygote, a blastomere, or a morula, blastocyst.

### DEFINITIONS

In the present invention, the following terms have the following meanings:

**"Embryonic cell"** refers to a diploid cell. Zygotes, blastomeres, morula and blastocysts are embryonic cells.

**"Gamete"** refers to a germinal cell for performing fertilization such as, for example, a sperm cell or an egg (oocyte).

**"Germinal cell"** refers to a haploid cell.

**"Holding pipette"** refers to a pipette configured to stabilize an oocyte, a germinal cell or an embryonic cell with gentle suction applied by a microinjector.

***"In vitro* procedure"** refers to an *in vitro* fecundation procedure. The *in vitro* procedure comprises steps from sampling of sperm cells and/or oocytes up to before the implantation of the embryo(s) resulting from the fecundation of oocyte(s) by sperm cell(s).

**"Mechanobiology parameter"** refers to a parameter measuring the influence of physical factors, such as force, on the cell by converting mechanical stimuli into biochemical signal.

**"Micropipette"** refers a thin hollow pipette configured to collect at least one sperm cell.

**"Pipette"** refers to a laboratory instrument that may be used to transport and manipulate fluids and cells. Pipette can also be referred to as pipet, micropipette, pipettor or dropper.

### BRIEF DESCRIPTION OF THE DRAWINGS

**Figure 1** is a representation of the system according to one embodiment of the invention wherein the pipette 100 is a holding pipette holding an oocyte 210 and comprising a strain gauge sensor 150.
**Figure 2** is a representation of the system according to one embodiment of the invention wherein the system comprises a vacuum generator 140 generating a suction force in the constriction channel 105 thereby deforming the oocyte 210.
**Figure 3** is a representation of the system according to one embodiment of the invention wherein the pipette 100 is a micropipette holding a sperm cell 210 and comprising a strain gauge sensor 150.
**Figure 4** is a representation of the system according to one embodiment of the invention wherein the sensor is a Raman spectrometer (151, 155) configured to generate a spectrum of the oocyte 210 held by a holding pipette 100.
**Figure 5** is a representation of the system according to one embodiment of the invention wherein the sensor is a Raman spectrometer (151, 155) configured to generate a spectrum of the sperm cell 210 held inside a micropipette 100.
**Figure 6** is a representation of the system according to one embodiment of the invention wherein the pipette 100 comprises a porous membrane 110 disposed transversally inside the pipette 100 and a negative pressure applicator 120 to secure the sperm cell 210 inside the pipette 100.
**Figure 7** is a representation of the system according to one embodiment of the invention wherein the sensor comprises two electrodes 130 disposed on the internal surface of the pipette 100 holding a sperm cell 210 between the two electrodes 130.
**Figure 8** is a representation of the system of Figure 7 wherein the electrodes 130 comprise an extremity disposed perpendicularly to the longitudinal axis of the pipette 100 to secure the sperm cell 210 inside the pipette 100.
**Figure 9** is a representation of the system according to one embodiment of the invention comprising a Petri dish 160 and wherein the sensor is a Raman spectrometer (151, 155) configured to generate a spectrum of the sperm 210 held by in the Petri dish 160.
**Figure 10** is a representation of the system according to one embodiment of the invention comprising a Petri dish 160 wherein an array of sensors 150 is disposed.
**Figure 11** is a block diagram representing schematically a particular embodiment of a computing unit 9 for assessing a germinal cell and/or an embryonic cell compliant with the present disclosure.

### DETAILED DESCRIPTION

This invention relates to a system for assessing a germinal cell 210 and/or an embryonic cell 210 in an *in vitro* procedure.

The germinal cell 210 is a haploid cell such as a gamete. The germinal cell 210 thus comprises only one set of chromosomes. For example, the germinal cell 210 is an oocyte or a sperm cell.

The embryonic cell 210 is a diploid cell. The embryonic cell 210 thus comprises two sets of chromosomes. The embryonic cell 210 is issued from the fusion of two haploid cells. For example, the embryonic cell 210 is formed by a fertilization event, preferably an *in vitro* fertilization, between two gametes. The embryonic cell 210 is defined in this disclosure as the early development stages of an embryo (or pre-embryo). The embryo may be at different early stages of development. For example, the embryo is a zygote at the earliest development stage, a blastomere produced by cell division (cleavage) of the zygote, a morula when the embryo contains 16 cells or a blastocyst when the embryo comprises an inner cell mass (embryoblast) and an outer layer of trophoblast cells (trophectoderm). The embryonic cell 210 refers to the whole embryo. The device of this disclosure allows to assess the whole living embryo and the surrounding media.

The *in vitro* procedure may be an *in vitro* fecundation procedure. The *in vitro* procedure may refer to at least one of the steps of the fecundation procedure from the sampling of gametes to the preparation of an embryo for the insertion into uterus. The *in vitro* procedure excludes all the *in vivo* steps so that there is no interaction with the body of a patient. The *in vitro* procedure may comprise intracytoplasmic sperm injection wherein a sperm cell is injected into an oocyte. The sperm cell and/or the oocyte used for the intracytoplasmic sperm injection are assessed using the system of this disclosure. The *in vitro* procedure may comprise natural *in vitro* fertilization wherein sperm cells are disposed in a dish with an oocyte, the sperm cells fecundating the oocyte naturally. The embryonic cell 210 assessed using the system of this disclosure may be issued from intracytoplasmic sperm injection or natural *in vitro* fertilization.

The system of this disclosure comprises:
- a pipette 100;
- at least one sensor 150; and
- at least one computing unit 9.

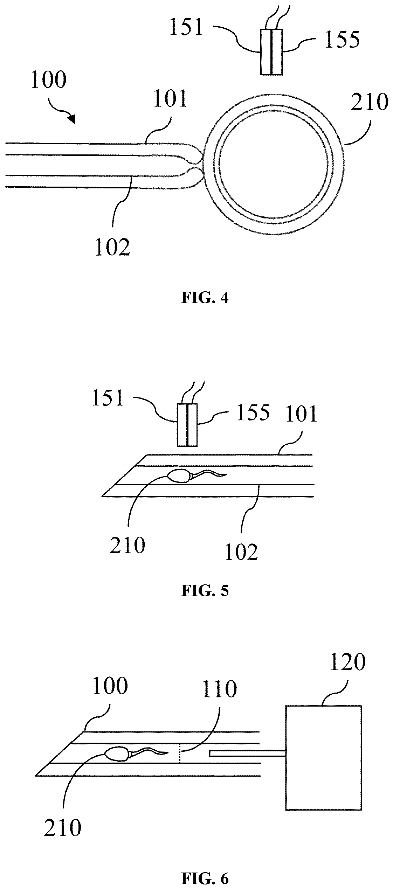

The pipette 100 is configured to hold at least one germinal cell 210 and/or an embryonic cell 210. The pipette 100 has a general cylindrical shape extending along a pipette axis. The pipette 100 is hollow, i.e., it has an internal cavity extending along the pipette axis. The pipette 100 thus has an external surface 101 and an internal surface 102 shown for example in Figure 4 and Figure 5.

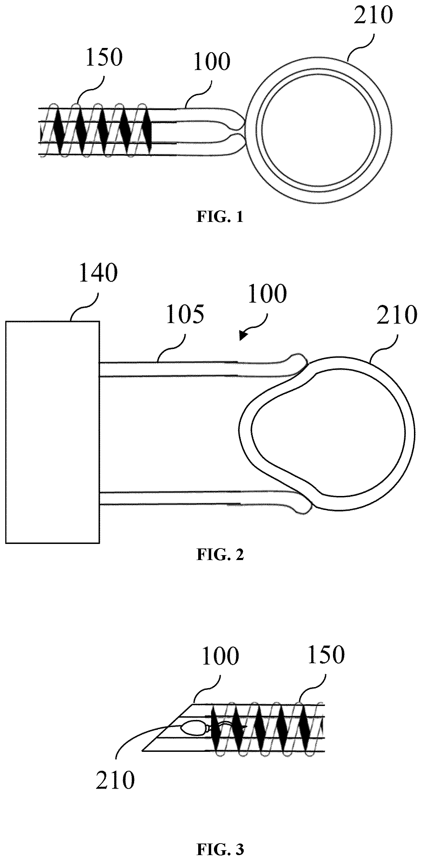

For example, the pipette 100 is a holding pipette as represented in Figure 1, Figure 2 and Figure 4. The holding pipette preferably has at its first extremity, a rounded tip and polished opening as to not damage the germinal cell 210 and/or an embryonic cell 210 held in contact with the tip for example using a gentle suction applied by a microinjector. The holding pipette may have an outer diameter measured perpendicularly to the pipette axis ranging from 100 µm to 150 µm. The holding pipette may have a total length measured parallelly to the pipette axis ranging from 50 mm to 60 mm. The inner diameter of the holding pipette is the diameter of the internal cavity measured perpendicularly to the pipette axis. The internal diameter may range from 20 µm to 200 µm. This allows to have an internal diameter which is lower than or equal to the size of the germinal cell 210 and/or an embryonic cell 210.

For example, the pipette 100 is a micropipette as represented in Figure 3, Figure 5, Figure 6, Figure 7 and Figure 8. The micropipette may be part of a microelectromechanical system (MEMS). The micropipette preferably has at its first extremity a sharp tip, with or without spike. The micropipette may have an outer diameter measured perpendicularly to the pipette axis ranging from 10 µm to 20 µm. The micropipette may have a total length measured parallelly to the pipette axis ranging from 50 mm to 60 mm. The sharped tip measured parallelly to the pipette axis may range from 18 mm to 22 mm. The inner diameter of the micropipette is the diameter of the internal cavity measured perpendicularly to the pipette axis. The internal diameter may range from 5 µm to 7 µm. This allows to have an internal diameter which is larger than the size of s sperm cell 210. Indeed, the micropipette is configured to held the germinal cell 210 or the embryonic cell 210 inside the internal cavity. Therefore, the internal surface 102 is preferably configured to be in contact with the germinal cell 210 or the embryonic cell 210.

In the embodiment wherein the germinal cell 210 is a gamete, the pipette 100 may be configured to put the gamete held by the pipette 100 in contact with another gamete for intracytoplasmic sperm injection. For example, the pipette 100 is a micropipette containing a sperm cell to be assessed and is configured to inject the assessed sperm cell inside an oocyte. In another example, the pipette 100 is a holding pipette holding an oocyte to be assessed and the pipette 100 is configured to inject a sperm cell in the assessed oocyte. In another example, the system comprises two pipettes 100: the first pipette 100 is a micropipette containing a sperm cell to be assessed while the second pipette 100 is a holding pipette holding an oocyte to be assessed; the first pipette 100 being configured to inject the assessed sperm cell in the assessed oocyte held by the second pipette 100.

The pipette 100 may comprise a constriction channel 105. The constriction channel 105 is a part of the internal cavity of the pipette 100 wherein the inner diameter is lower compared to the other part of the internal cavity. Equivalently other internal holding structures could be integrated (pins, flatten section of the glass capillary). For example, in Figure 2, the tip of the pipette 100 in contact with the cell 210 has an inner diameter larger than the rest of the pipette 100. Said rest of the pipette 100 thus forms the constriction channel 105. The constriction channel 105 is advantageous to secure a germinal cell 210 or an embryonic cell 210 inside the pipette to avoid losing the cell 210 during the analysis.

The pipette 100 may comprise, as represented in Figure 6, a porous membrane 110 disposed transversally, i.e., substantially perpendicularly to the pipette axis, inside the pipette 100 and a negative pressure applicator 120 configured to decrease a pressure inside the pipette 100 thereby generating a low-pressure in the internal cavity. The negative pressure applicator 120 thus allows to provide a pressure inside the pipette lower than the external pressure. This advantageously allows to secure a germinal cell 210 or an embryonic cell 210 inside the pipette to avoid losing the cell 210 during the analysis. The porous membrane 110 prevents the movement of the cell 210 towards the second extremity (opposed to the first extremity) of the pipette 100.

The sensor 150 is configured to measure a physical parameter of the germinal cell 210 and/or embryonic cell 210 held by the pipette 100.

The sensor 150 may be a strain gauge sensor disposed on the external surface 101 of the pipette 100 as represented in Figure 1 and Figure 3. The physical parameter measured by the strain gauge sensor is a strain of the germinal cell 210 and/or embryonic cell 210. In other words, the strain gauge sensor is configured to measure the amount of strain or deformation in germinal cell 210 and/or embryonic cell 210 when it is subjected to an external force or load. For example, the germinal cell 210 and/or embryonic cell 210 may be deformed by applying a positive pressure on the surface of the cell towards the inside of the cell. In another example, the germinal cell 210 and/or embryonic cell 210 may be deformed by applying a negative pressure on the surface of the cell implying a deformation of the surface of the cell towards the outside of the cell. The sensor 150 may be configured to apply said pressure on the surface of the cell 210. The pipette 100 may comprise a spring which compresses and expends according to the deformation of the cell. The strain may be determined by measuring a current variation in the spring. In another example, the inductive or capacitive change due to the spring deformation alter the inductance or capacitance in a nearby coil or plate system; the measure of the inductance or capacitance thus leads to the determination of the strain. In another example, a strain gauge could be attached to the deformable spring and the change can be measured by a Wheastone bridge circuit to accurately quantify the applied force. In another example, an image of the deformed cell may be received by an imaging system such as the microscope used for the ICSI procedure. Alternately, one can also do image processing using the same ICSI microscope camera, o measure the inductive or capacitive change by the spring deformation, which will alter the inductance or capacitance in a nearby coil or plate system.

In another embodiment compatible with the previous embodiment and represented in Figure 3, the sensor 150 may be configured to apply a variation of pressure on a surface of the germinal cell 210 and/or embryonic cell 210 and to measure the elastic deformation of said surface, i.e., the reversible change in shape or size of the cell 210 when the pressure is applied and subsequently removed. Elastic deformation may be measured by imaging using a microscope. For example, a set of images are recorded using a microscope for a set of predetermined pressures. Variation of the deformation may thus be determined according to the variation of pressure. Mechanobiology parameters such as Young modulus, metabolites, ribonucleic acid (RNA), expressed proteins, deoxyribonucleic acid (DNA) integrity, presence of reactive species, deformability of the cell membrane, morphology dynamics, stiffness and elasticity of the cell which indicate properties of the zona pellucida, viscoelastic properties such as cytoskeletal integrity, overall size of the cell, zona pellucida hardening which can affect embryo hatching and reduce implantation rates, or osmotic pressure of the assessed germinal cell 210 and/or embryonic cell 210 may be determined from the measured elastic deformation. The pressure may be a positive pressure deforming the surface of the cell towards the inside of the cell. The pressure may be a negative pressure deforming the surface of the cell towards the outside of the cell.

For the two previous embodiments of the sensor 150, the negative pressure may be generated by a vacuum generator 150. The sensor 150 may comprise said vacuum generator 150. The negative pressure may be applied inside the constriction channel 105 of the pipette 100 therefore leading to a suction force inside the constriction channel 105 as represented in Figure 2.

For the two previous embodiments of the sensor 150, the positive pressure may be applied using a microspring in contact with the surface of the held germinal cell 210 and/or embryonic cell 210. The microspring may be disposed inside the internal cavity or outside the pipette 100. The microspring is advantageous since it also provides mechanical stimulation of the oocyte which improves quality of fecundation.

For the two previous embodiments of the sensor 150, the positive pressure may be applied using a surface acoustic wave generator. The surface acoustic wave generator is configured to generate acoustic waves. The acoustic waves propagate and reflect on the germinal/embryonic cells 210. A measure of the reflected acoustic waves allows to determine the strain or elastic deformation of the cell 210. Indeed, the acoustic impedance of the acoustic waves, the reflection of the acoustic waves, the transmission of the acoustic waves and/or the resonance of acoustic scattering may be measured. This allows to determine properties such as stiffness and elasticity, density variations and viscoelastic properties.

The surface acoustic wave generator is advantageous since it also provides mechanical stimulation of the oocyte which improves quality of fecundation. The surface acoustic wave generator may be disposed inside the internal cavity or outside the pipette 100.

In another embodiment of the sensor 150 compatible with the previous embodiments and represented in Figure 4 and Figure 5, the sensor 150 is a Raman spectrometer comprising a light source 151 configured to emit a probe light beam towards the germinal cell 210 and/or embryonic cell 210, and a microdetector 155 configured to detect a light beam received from the cell 210 in response to the probe light beam. The light source 151 is preferably disposed outside the pipette 100. The light source 151 may be the light source of the microscope used for the ICSI procedure. The microdetector 155 is preferably disposed outside the pipette 100. The light source 151 and/or the microdetector 155 can be oriented along or across the pipette 100 or can be coupled to the microscope used for the ICSI procedure allowing in-plane detections. In the embodiment wherein the cell 210 is held inside the internal cavity, the curvature of the pipette 100 advantageously allows to collimate the probe light beam. The physical parameter measured by the sensor 150 is a spectrum of the detected light beam. The pipette 100 may comprise gold nanoparticles preferably disposed on the internal surface 102 at a position wherein the cell 210 is held so that the sensor 150 is configured to perform Surface Enhanced Raman spectroscopy. The pipette 100 may comprise lenses such as reflective lateral lenses such as rolled-up metallic foil achieving prepatterned microtubes or a reflective coating disposed on the internal surface 102 at a position wherein the cell 210 is held. The lenses advantageously allow to enhance reflection of the light beam so as to improve the signal-to-noise ratio of the spectrum. Raman spectroscopy allows to assess cell membrane integrity and DNA intactness with high sensitivity before fecundation.

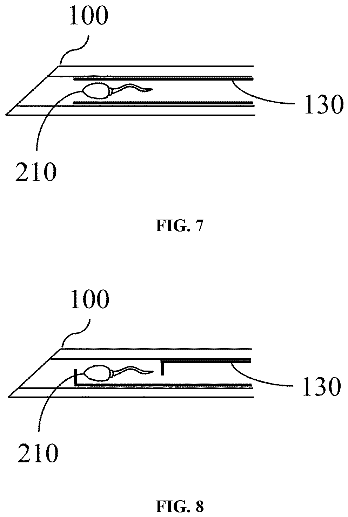

In another embodiment of the sensor 150 compatible with the previous embodiments and represented in Figure 7 and Figure 8, the sensor 150 comprises two electrodes 130 disposed on the internal surface 102 of the pipette 100. The electrodes 130 may be metal layer having a thickness ranging from 10 nm to 100 nm. The electrodes 130 may be in the form of a rolled-up tube placed on the internal surface of the pipette 100. Preferably, the pipette 100 is configured to hold a sperm cell 210 or embryonic cell 210 so that the cell 210 is positioned between the two electrodes 130. The physical parameter measured by the sensor 150 may be an impedance of the sperm cell 210 or embryonic cell 210. To do so, a voltage is applied to the electrodes 130. The current flowing between the two electrodes is then measured. Said current depends on the impedance of the sperm cell 210 or embryonic cell 210. Electrodes 130 are advantageous because, when comprising conductive material, they may also serve as lenses for Raman spectroscopy. In the embodiment represented in Figure 8, electrodes 130 comprise an extremity disposed substantially perpendicularly to the longitudinal axis of the pipette 100. This advantageously allows to secure the cell 210 inside the pipette 100. The extremities may be selectively angled to facilitate the passage of the cell 210 inside the pipette 100.

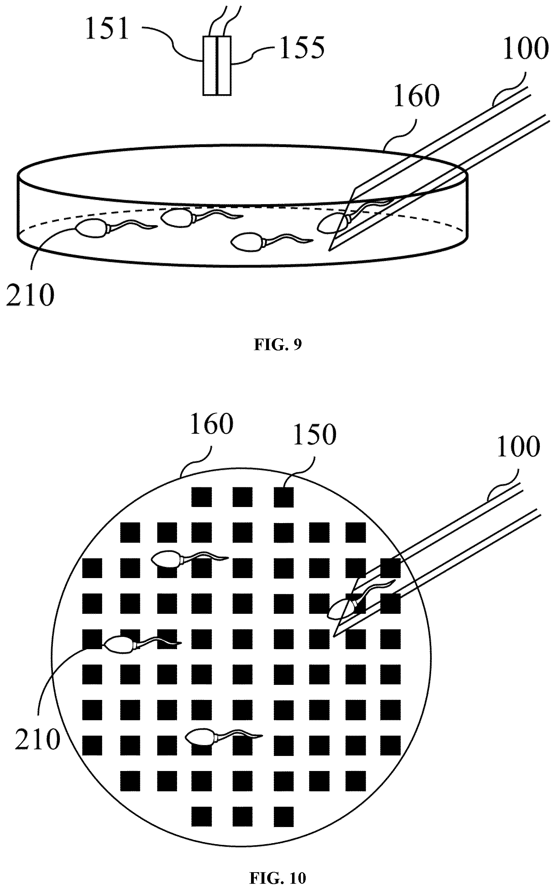

As represented in Figure 9 and Figure 10, the system may further comprise a petri dish 160 configured to contain at least one germinal cell 210 and/or an embryonic cell 210, the petri dish 160 comprising at least one sensor 150 as described above. The sensor 150 for the assessment of the germinal cell 210 and/or embryonic cell 210 disposed in the petri dish 160 may be a Raman spectrometer, for example the Raman spectrometer described above as represented in Figure 9. The sensor 150 for the assessment of the germinal cell 210 and/or embryonic cell 210 disposed in the petri dish 160 may comprise at least one electrode, preferably a plurality of electrodes disposed in array on the bottom surface of the petri dish 160 as represented in Figure 10. When a voltage is applied to the electrodes, the current flowing between two electrodes is measured. Said current depends on the impedance of the sperm cell 210 or embryonic cell 210. Electrodes are advantageous because, when comprising conductive material, they may also serve as lenses for Raman spectroscopy. The sensor 150 for the assessment of the germinal cell 210 and/or embryonic cell 210 disposed in the petri dish 160 ma comprise at least one nanoscale spring system with a strain response to piconewton-scale forces. The germinal cell(s) 210 and/or embryonic cell(s) 210 disposed in the petri dish 160 may undergo a first assessment before being held by the pipette 100 for further assessment. This allows to increase the number of assessments or speed-up the screening of them prior their pick up by the pipette.

The computing unit 9 is configured to assess, based on the physical parameter measured by the sensor 150, the germinal cell 210 and/or embryonic cell 210.

When the sensor 150 is configured to measure the elastic deformation of the surface of the germinal cell 210 and/or embryonic cell 210, the computing unit 9 may be configured to compute a mechanobiology parameter, such as Young modulus, metabolites, RNAs, expressed proteins, DNA integrity, presence of reactive species, deformability of the cell membrane, morphology dynamics, stiffness and elasticity of the cell which indicate properties of the zona pellucida, viscoelastic properties such as cytoskeletal integrity, overall size of the cell, zona pellucida hardening which can affect embryo hatching and reduce implantation rates, or osmotic pressure of the assessed germinal cell 210 and/or embryonic cell 210, from the measured elastic deformation. The determination of mechanobiology parameter advantageously provides insights into the stiffness of the cells 210. The stiffness of the cells 210 is linked to the quality of the assisted fertilization involving the assessed cell 210.

When the sensor 150 is a Raman spectrometer, the computing unit 9 may be configured to determine, from the measured spectrum, a DNA integrity of the germinal cell 210 and/or embryonic cell 210, a presence of oxidative stress markers in the germinal cell 210 and/or embryonic cell 210, information on biomolecules such as nucleic acids, protein, carbohydrates, and lipids inside the germinal cell 210 and/or embryonic cell 210, and/or mechanical properties of the germinal cell 210 and/or embryonic cell 210. This assessment reflects cellular genotypes, phenotypes and physiological states of the assessed cell 210.

When the sensor 150 comprises electrodes 130, the computing unit 9 may be configured to compute, from the measured impedance, integrity of the sperm cell 210 or embryonic cell 210 or environmental factors such as micro RNAs, metabolites, expressed proteins in the surrounding media (also named here environmental factors). This allows to determine oxidative stress, expressed biomarkers, and also some properties on the membrane and cytoplasm conductivity.

The computing unit 9 corresponds for example to a workstation, a laptop, a tablet, a smartphone, or a head-mounted display (HMD).

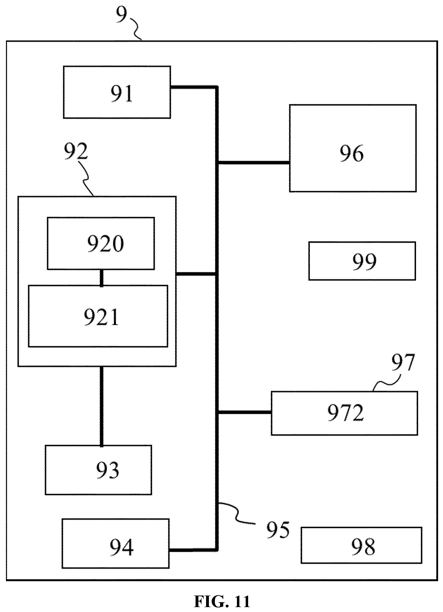

That computing unit 9 may comprise at least one of the following elements schematically represented in Figure 11, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the computing unit 9.

The computing unit 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card, for example for obtaining spectra of the Raman spectroscopy or images of the deformation of the cell 210. According to a variant, a display device is external to computing unit 9 and is connected thereto by a cable or wirelessly for transmitting the display signals. The computing unit 9, for example through the graphics card 92, comprises an interface for transmission or connection adapted to transmit a display signal to an external display means such as for example an LCD or plasma screen or a video-projector. In this respect, the RF unit 99 can be used for wireless transmissions. As will be understood by a skilled person, the presence of the graphics card 92 is not mandatory, and can be replaced with entire CPU processing and/or simpler visualization implementations.

The RAM 97 may comprise a computer program product 972 comprising instructions which, when the program is executed by the system, cause the computing unit 9 to carry out assessment of the germinal cell 210 and/or the embryonic cell 210.

When switched-on, the microprocessor 91 loads and executes the instructions of the program 972 contained in the RAM 97.

The invention further relates to a method for assessing a germinal cell 210 and/or an embryonic cell 210 in an *in vitro* procedure. The method may be applied before, during or after natural *in vitro* fertilization. The method may be applied before, during or after intracytoplasmic sperm injection.

The method comprises providing a system comprising a pipette 100, at least one sensor 150 and at least one computing unit 9. Said system may be the system in one of the embodiments described hereabove.

At least one germinal cell 210 and/or at least one embryonic cell 210 to be assessed is provided. The cell 210 may be prepared before assessment. For example, the tail of the sperm cell may be cut. The cell 210 is then hold using the pipette 100. The cell is either held inside the internal cavity of the pipette 100 or held in contact with the tip of the pipette 100 by gentle suction applied by a microinjector.

The method comprises a step of measuring a physical parameter of the germinal cell 210 and/or the embryonic cell 210 with the sensor 150.The physical parameter may be a strain of the cell 210, an elastic deformation of the surface of the cell 210, a Raman spectrum of a light reflected by the cell 210, an impedance of the cell 210 or a combination thereof. The physical parameter may be measured according to the different embodiments of the sensor 150 as described above.

The method comprises a step of assessing, based on the measured physical parameter, the at least one germinal cell 210 and/or an embryonic cell 210. The method may assess:
- a mechanobiology parameter, preferably Young modulus, of the cell 210 from the measured elastic deformation;
- a DNA integrity of the cell 210, a presence of oxidative stress markers in the cell 210, information on biomolecules such as nucleic acids, protein, carbohydrates, and lipids inside the cell 210, and/or mechanical properties of the cell 210 from the Raman spectrum; and/or
- integrity of the cell 210 or environmental factors from the measured impedance.

The assessment of gametes or embryonic cells may allow to predict pregnancy viability after intracytoplasmic sperm injection of the sperm cell in the oocyte. The prediction of the pregnancy outcomes may be performed by the computing unit 9. Indeed, the implantation and live birth rates of euploid embryos (embryos with the correct number of chromosomes) are significantly higher than those of aneuploid embryos. The method and device of this disclosure is able to assess embryos to determine if the embryo is euploid.

### NUMERICAL REFERENCES

100 - Pipette // 101 - External surface // 102 - Internal surface // 105 - Constriction channel // 110 - Porous membrane // 120 - Negative pressure applicator // 140 - vacuum generator // 150 - Sensor // 151 - light source of Raman spectrometer // 155 - microdetector of Raman spectrometer // 160 - Petri dish // 210 - germinal cell or embryonic cell.

## Claims

1. A system for assessing a germinal cell (210) and/or an embryonic cell (210) in an *in vitro* procedure, the system comprising
- a pipette (100) configured to hold at least one germinal cell (210) and/or an embryonic cell (210);
- at least one sensor (150) configured to measure a physical parameter of the germinal cell (210) and/or embryonic cell (210);
- at least one computing unit (9) configured to assess, based on the physical parameter, the germinal cell (210) and/or embryonic cell (210).

2. The system according to claim 1 wherein the sensor (150) is a strain gauge sensor disposed on an external surface (101) of the pipette (100) and the physical parameter is a strain of the germinal cell (210) and/or embryonic cell (210).

3. The system according to claim **1** or **2** wherein the sensor (150) is configured to apply pressure on a surface of the germinal cell (210) and/or embryonic cell (210) and the physical parameter is the elastic deformation of said surface, the computing unit (9) being configured to compute a mechanobiology parameter, preferably Young modulus, from the measured elastic deformation.

4. The system according to claim **3** wherein the sensor (150) comprises a microspring, and/ or a surface acoustic wave generator.

5. The system according to claim **3** or **4** wherein the pipette (100) comprises a constriction channel and the system further comprises a vacuum generator (140) configured to generate a suction force in the constriction channel.

6. The system according to any one of claims **1** to **5,** wherein the sensor (150) is a Raman spectrometer comprising a light source (151) configured to emit a probe light beam towards the germinal cell (210) and/or embryonic cell (210), and a microdetector (155) configured to detect a light beam received from the germinal cell (210) and/or embryonic cell (210) in response to the probe light beam, the physical parameter being a spectrum of the detected light beam, the computing unit (9) being configured to determine a DNA integrity of the germinal cell (210) and/or embryonic cell (210), a presence of oxidative stress markers in the germinal cell (210) and/or embryonic cell (210), information on biomolecules inside the germinal cell (210) and/or embryonic cell (210), and/or mechanical properties of the germinal cell (210) and/or embryonic cell (210) from the spectrum.

7. The system according to claim **6** wherein the pipette (100) comprises gold nanoparticles so that the sensor (150) is configured to perform Surface Enhanced Raman spectroscopy.

8. The system according to any one of claims **1** to **7** further comprising a petri dish configured to contain at least one germinal cell (210) and/or an embryonic cell (210), the petri dish comprising the at least one sensor (150).

9. The system according to any one of claims **1** to **8** wherein the germinal cell (210) is a gamete, the pipette (100) being configured to put the gamete held in the pipette (100) in contact with another gamete for intracytoplasmic sperm injection.

10. The system according to any one of claims **1** to **9** wherein the pipette (100) is a holding pipette.

11. The system according to any one of claims **1** to **9** wherein the pipette (100) is a micropipette.

12. The system according to claim **11** wherein the pipette (100) is configured to hold a sperm cell and comprises a constriction channel and/or a porous membrane (110) disposed transversally inside the pipette (100) and a negative pressure applicator (120) configured to decrease a pressure inside the pipette (100).

13. The system according to claim **11** or **12** wherein the sensor (150) comprises two electrodes (130) disposed on an internal surface (102) of the pipette (100) configured to hold a sperm cell so that the sperm cell is positioned between the two electrodes (130), the physical parameter being an impedance of the sperm cell, the computing unit (9) being configured to compute integrity of the sperm cell from the measured impedance.

14. A method for assessing a germinal cell (210) and/or an embryonic cell (210) in an *in vitro* procedure, the method comprising:
- providing a system comprising:
∘ a pipette (100);
∘ at least one sensor (150) configured to measure a physical parameter of a germinal cell (210) and/or an embryonic cell (210);
∘ at least one computing unit (9) configured to assess, based on the physical parameter, the germinal cell (210) and/or embryonic cell (210);
- providing at least one germinal cell (210) and/or at least one embryonic cell (210);
- holding the germinal cell (210) and/or the embryonic cell (210) using the pipette (100);
- measuring a physical parameter of the germinal cell (210) and/or the embryonic cell (210) with the sensor (150); and
- assessing, based on the physical parameter, the at least one germinal cell (210) and/or an embryonic cell (210).

15. The method according to claim **14** wherein the germinal cell (210) may be a sperm cell or an oocyte and the embryonic cell (210) may be a zygote, a blastomere, or a morula, blastocyst.
